# EUROPEAN PATENT SPECIFICATION

(11) **EP 4 319 640 B1**
(45) Date of publication and mention of the grant of the patent: **04.06.2025**
(21) Application number: 22718459.5
(22) Date of filing: 01.04.2022
(51) Int. Cl.: A61B 6/00, A61B 6/04, A61B 6/58

(54) **PERSONALIZED CRITICAL CARE IMAGING**
PERSONALISIERTE BILDGEBUNG IN DER INTESIVPFLEGE
IMAGERIE PERSONNALISÉE DE SOIN INTENSIF

(30) Priority: 05.04.2021 US 202163170594 P
(43) Date of publication of application: 14.02.2024
(73) Proprietor: Carestream Health, Inc., Rochester, NY 14608 (US)
(72) Inventor: FOOS, David H., Rochester, New York 14608 (US); SIEGEL, Eliot L., Rochester, New York 14608 (US); DAMANY, Dharmendu, Rochester, New York 14608 (US); BOGONI, Luca, Rochester, New York 14608 (US)
(74) Representative: Pritzlaff, Stefanie Lydia
(86) International application number: PCT/US2022/022975
(87) International publication number: WO 2022/216532

(56) References cited:
- WO-A1-2021/141681
- US-A1- 2015 216 450
- US-A1- 2015 265 219
- US-A1- 2016 051 216
- US-A1- 2020 251 206

## Description

### TECHNICAL FIELD

The disclosure relates generally to digital radiographic imaging of the human anatomy and more particularly to solutions for improving patient care and treatment protocol in critical care situations according to monitoring of image contents over time.

### BACKGROUND

To maintain high standards of care for the patient in an intensive care unit (ICU), change assessment, derived from ongoing, longitudinal monitoring of the patient's health status, provides a useful basis for caregiver assessment of, and refinement to, treatment protocol. There are a number of components to longitudinal monitoring of patients in the ICU, including vital signs monitoring, tracking symptoms such as blood pressure, heartrate (EKG), and breathing patterns, for example. Additionally, histological and imaging data, as well as visual and audio cues, can be integral to effective health status monitoring.

Imaging using x-rays, in particular, can be an effective tool in change assessment of a patient's condition. In many instances, for example, patients in the Intensive Care Unit (ICU) who suffer from severe cardio-pulmonary disfunction, such as due to pneumonia or heart failure, are routinely monitored using chest x-rays. Chest x-ray images allow the attending team to evaluate disease progression or response to a course of treatment, as evidenced by lung opacifications. Clinical assessment of changes in the lungs can be obtained by comparing the current x-ray with a recent prior x-ray. As part of this assessment, the clinician can observe changes in opacification between most current and next recent images, and judge how much change there is in the patient condition, with image content evaluated to indicate improvement, worsening, or relative stability. For instance, imaging with x-rays provides indication of fluid levels in the lungs, and thus helps in tracking fluid level changes, such as in comparison between newly acquired images and prior images.

Treatment refinement is an intrinsically reactive process, as progression or recession of disease, such as can be evidenced by fluid level increase or decrease within the lungs, can be determined based on an assessment of (or change in) the patient condition in response to the given treatment. Treatment refinements may include drug dosage changes, insertion or removal of catheters, and refinements of respirator settings, for example.

X-ray imaging in the contemporary ICU is performed by radiographic technologists using mobile x-ray imaging devices, e.g., mobile digital radiography (mDR) systems, such as the DRX Revolution Mobile Imaging System from Carestream Health, Rochester, NY. A key factor in assessing changes in patient health based on x-ray images relates to the consistency of image capture parameters between a newly acquired x-ray image and a prior x-ray image or set of prior x-ray images. For instance, when comparing images, it is useful to know whether or not the same exposure techniques were followed, whether the same patient positioning and acquisition geometry were used, and, for chest x-ray images, whether the acquisition was performed with the patient in the same phase of a breathing cycle. Inconsistencies in capture geometry and image processing parameters can make it challenging to differentiate changes in image acquisition factors from actual changes in patient health condition.

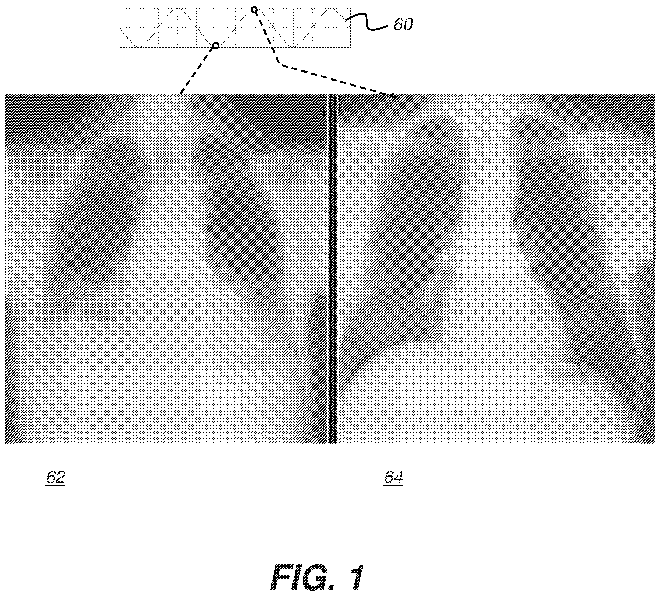

By way of example, the two x-ray images 62, 64 in FIG. 1 were obtained from the same patient at different phases of the breathing cycle, which breathing cycle is represented by a graph 60, where full inspiration is indicated at a peak of the cycle (e.g. image 64) and full expiration is indicated at a trough of the cycle (e.g. image 62), making it difficult to identify incremental changes in patient condition. There is no feasible mechanism to register the images of the lung fields in these two images, thus, changes due to differences in patient condition are confounded with differences caused by image capture variations.

While recently obtained chest x-ray images offer great benefits to the clinician, there are a number of factors with negative impact on evaluation accuracy. These can include:
(i) Inconsistent x-ray acquisition geometry. The acquisition geometry for a radiographic image involves the relative alignment of the x-ray tube, the patient, and the x-ray sensing detector. Positioning differences increase the difficulty of comparing current x-ray images against a prior x-ray image. For example, the projection of features such as support tubes onto the imaging detector can be highly dependent on 3D geometrical alignment of the patient relative to the x-ray tube and digital imaging detector. When comparing two chest x-ray images of the same subject taken at different times, positioning differences relating to components of the acquisition system can lead to difficulties in relating anatomic structures to each other and can make it difficult to ascertain the relative arrangement of tubing and support hardware features to the corresponding patient anatomy.
(ii) Differences in x-ray acquisition technique. Differences in technique settings, such as set energy (kVp) and intensity (mAs) levels of the emitted x-ray beam, can significantly change the appearance of conditions such as pneumonia or pulmonary edema and lung disease in general.
(iii) Disparity in respiratory phase. Chest x-ray images can be obtained at different phases of respiration, such as full inspiration, full expiration, or some intermediate phase. Accurate assessment of lung opacification can require image acquisition at full inspiration, otherwise spurious basal opacities can cause false positive findings. Pneumothoraces, on the other hand, are more conspicuous where images are acquired at full expiration.
(iv) Grayscale variability. Variations in grayscale presentation can confound accurate assessment for x-ray images of the same subject taken at different times or using different equipment. Inconsistent grayscale presentation in chest x-rays complicates the ability to assess changes in lung opacification by the clinician. Grayscale variability can also cause difficulties for automatic image assessment by a software algorithm.

It can be appreciated that there is a pressing need for methods that address problems that confound proper interpretation and use of x-ray images taken at different times, particularly for chest x-rays in the ICU environment. Further, there is a need for appropriate scheduling of x-ray acquisition that is based on up-to-date information on the condition of the ICU patient and assists the clinician to maintain a suitable level of patient monitoring using x-ray imaging systems.

US 2015 / 265 219 A1 discloses a method for adapting a medical system to an object movement during medical examination of the object and a medical system configured for carrying out the method. The medical system has a device for detecting and quantifying a motion of the object before or during an acquisition of diagnostic data. The system for detecting and quantifying a motion of the object identifies and qualifies the occurrence of object motion and to automatically suggest an adaptation of the diagnostic data acquisition strategy/technique as a function of the object motion.

US 2015 / 216 450 A1 relates to a patient monitor for detecting patient movement or abnormal breathing. Images of a patient are obtained by a stereoscopic camera.

### SUMMARY

In accordance with the present invention, a method as set forth in independent claim 1 is provided. Preferred embodiments of the invention are claimed in the dependent claims. An object of the present disclosure is to address the need to maintain more effective ongoing monitoring of patient condition using consistent radiographic imaging factors and appropriate timing based on changes in the patient condition.

According to an embodiment of the present disclosure, there is provided a system and method to improve the consistency of capture of x-ray images of the same patient in an ICU or other critical care facility at different moments in time. According to one aspect, a plurality of radiographic images of a patient are captured using a common geometric relationship with respect to an x-ray source, the patient and a digital detector. A change in the patient's health is assessed based on the radiographic images of the patient and a time to capture a next radiographic image of the patient is automatically scheduled, using the common geometric relationship.

These objects are given only by way of illustrative example, and such objects may be exemplary of one or more embodiments of the invention. Other desirable objectives and advantages inherently achieved may occur or become apparent to those skilled in the art. The invention is defined by the appended claims.

### BRIEF DESCRIPTION OF THE DRAWINGS

The foregoing and other objects, features, and advantages of the invention will be apparent from the following more particular description of the embodiments of the invention, as illustrated in the accompanying drawings. The elements of the drawings are not necessarily to scale relative to each other.
FIG.1 shows exemplary x-ray images obtained from the same patient during different phases of the respiratory cycle.
FIG. 2 is a schematic diagram that shows an exemplary arrangement of imaging components that can be used to form a radiographic imaging apparatus.
FIG. 3 is a logic flow diagram that shows a sequence for radiographic imaging that applies steps for improved consistency and can generate subsequent exam scheduling.
FIG. 4 is a timing chart that shows a patient breathing cycle.
FIG. 5 shows an exemplary operator interface screen with a displayed reminder prompt.

### DETAILED DESCRIPTION OF THE EMBODIMENTS

This application claims priority to US 63/170,594, provisionally filed on April 5, 2021, entitled "PERSONALIZED CRITICAL CARE IMAGING" in the names of Foos et al.

The following is a detailed description of the preferred embodiments, reference being made to the drawings in which the same reference numerals identify the same elements of structure in each of the several figures.

Various relative terms such as "above," "below," "top," "bottom," "height," "depth," "width," and "length," etc. may be used in the present disclosure to facilitate description of various embodiments. The relative terms are defined with respect to a conventional orientation of a structure and do not necessarily represent an actual orientation of the structure in manufacture or use. The following detailed description is, therefore, not to be taken in a limiting sense.

Where they are used, the terms "first", "second", and so on, do not necessarily denote any ordinal or priority relation, but may be used for more clearly distinguishing one element or time interval from another.

As used herein, the term "energizable" relates to a device or set of components that perform an indicated function upon receiving power and, optionally, upon receiving an enabling signal. The opposite state of "energizable" is "disabled".

The term "actuable" has its conventional meaning, relating to a device or component that is capable of effecting an action in response to a stimulus, such as in response to an electrical signal, for example.

The term "modality" is a term of art that refers to types of imaging. Modalities for a radiographic imaging system may be conventional x-ray, serial radiography or video x-ray, fluoroscopy, tomosynthesis, tomography, dual-energy, or other types of imaging that employ ionizing radiation directed through patient anatomy to a detector. Thus, references herein to "radiographic imaging" and "radiographic imaging apparatus" can apply for conventional x-ray, serial radiography, video x-ray, fluoroscopy, tomosynthesis, tomography, dual-energy, or other types of imaging using x-ray energy. The term "subject" refers to the patient who is being imaged and, in optical terms, can be considered equivalent to the "object" of the corresponding imaging system.

In the context of the present disclosure, the term "coupled" is intended to indicate a mechanical association, connection, relation, or linking, between two or more components, such that the disposition of one component affects the spatial disposition of a component to which it is coupled. For mechanical coupling, two components need not be in direct contact, but can be linked through one or more intermediary components or fields.

It will be understood that when an element is referred to as being "connected," or "coupled," to another element, it can be directly connected or coupled to the other element or intervening elements or magnetic fields may be present. In contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The term "set", as used herein, refers to a non-empty set, as the concept of a collection of elements or members of a set is widely understood in elementary mathematics. The term "subset", unless otherwise explicitly stated, is used herein to refer to a non-empty proper subset, that is, to a subset of the larger set, having one or more members. For a set S, a subset may comprise the complete set S. A "proper subset" of set S, however, is strictly contained in set S and excludes at least one member of set S.

The term "exemplary" indicates that the description is used as an example, representative of general nature or behavior, rather than implying that it is an ideal.

The term "in signal communication" as used in the application means that two or more devices and/or components are capable of communicating with each other in at least one direction via signals that travel over some type of signal path. Signal communication may be wired or wireless. The signals may be communication, power, data, or energy signals which may communicate information, power, and/or energy from a first device and/or component to a second device and/or component along a signal path between the first device and/or component and second device and/or component. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

Embodiments of the present disclosure help to enable personalized imaging for the ICU patient, taking advantage of information that may be routinely obtained in order to more efficiently schedule and use x-ray modalities and to help limit radiation exposure according to information specifically obtained from a patient.

Improving image capture and processing consistency facilitates accurate visual and analytical (quantitative) change assessment of a patient's health. Furthermore, consistent image capture and processing helps to enable an automated, or assisted sequence for scheduling or triggering capture of a new x-ray image of the patient, based on ongoing or "longitudinal" tracking of patient condition using image content. According to an embodiment of the present disclosure, changes in patient condition can be predicted by trending change vectors that are derived from multiple x-ray images captured consistently and, further, from combinations of x-ray images with other data, such as vital signs metrics, that may be available. Further, mathematical models derived from trending data can be utilized to determine if and when a subsequent image of the patient should be acquired. For example, if the mathematical model, derived using the x-ray images alone, or using images coupled with ancillary data, indicates a rapid or steep decline in respiratory performance, a subsequent image may be indicated and, according to an embodiment of the present disclosure, captured automatically or prompted at a predetermined point in time or at a predetermined respiratory phase. Conversely, if the trending model indicates that the patient is improving or indicates a stable condition, subsequent x-ray imaging may be scheduled or advised at a later time, depending on the analysis. The aforementioned sequence represents, by way of example, a method for optimizing the radiation dose delivered to the patient and, at the same time, optimizing image and information capture factors for providing effective treatment and care to the patient.

Consistent and precise image capture and processing may be difficult using mDR. According to an embodiment of the present disclosure, a new paradigm for ICU patient imaging can be leveraged to improve patient care, particularly in an ICU environment configured with mobile x-ray imaging equipment. The in-room x-ray equipment can be configured in a number of different ways that can enable highly consistent image capture. As was mentioned herein, consistent image capture can be used to help improve automated exam triggering. Consistency can be achieved through automated, remote-virtual, or technologist-assisted positioning of the x-ray tube and the collimator with respect to the patient anatomy to be imaged, e.g., the chest, and the imaging receptor, which is typically a flat panel digital radiography detector.

According to an embodiment of the present disclosure, automated, virtual, or technologist assisted positioning may be achieved using a stereoscopic visible light camera system mounted to an x-ray tube head, coupled with various fiducial markers, wherein the acquisition technique settings, such as kVp and mAs settings can be determined using a body mass index or other surrogate for patient anatomy thickness. It is understood that any other type of cameras that automatically computes a depth map can be used instead of a stereoscopic light camera. Other aspects of the imaging geometry can be configured in a manner that is consistent with prior images of the same patient. It is a further aspect of the present invention that the stereoscopic camera is used to precisely measure the phase of the respiratory cycle and thereby be used as one indication (trigger) for performing an exposure. Other methods for respiratory gating may be used as well, for instance, audio sensing or other sensing for detecting patient movement relating to the breathing cycle.

### Maintaining Consistent Image Capture

Embodiments of the present disclosure address the need for improved radiographic image consistency with solutions that can be applied over a broad range of image modalities. An area of particular need that can be addressed by employing solutions described herein relates to image consistency in the ICU and other critical care facilities in which periodic monitoring of patient condition includes repeated radiographic imaging sessions that capture images of the same anatomic region. Because the chest x-ray, as shown in the examples of FIG. 1, is widely used for ICU patients, embodiments described herein describe how solutions of the present disclosure can be implemented using the example of periodic chest x-rays. Embodiments of the present disclosure are not limited to chest x-rays, however, and other applications for patient conditions, anatomy, and imaging modes not specifically described herein can be apparent to those skilled in the radiographic imaging arts.

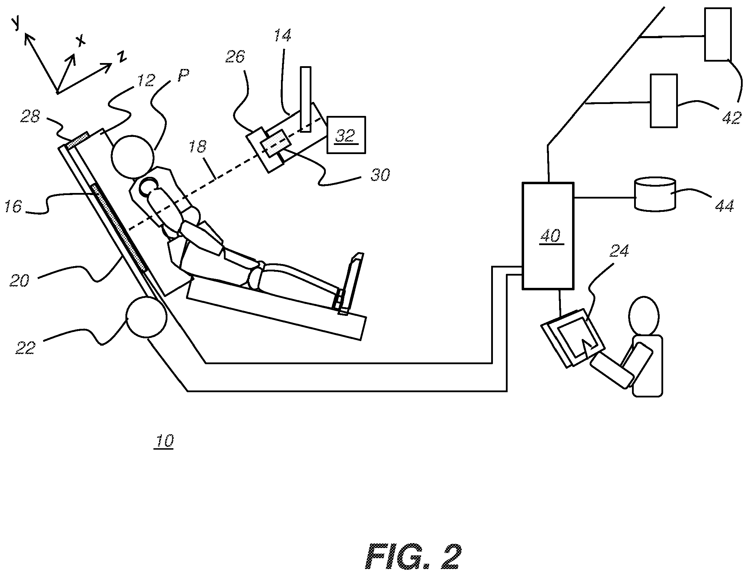

The schematic diagram of FIG. 2 shows an exemplary arrangement of imaging components that can be used to form a radiographic imaging apparatus 10 and can help to provide consistent imaging results. A patient P is disposed on a bed or other support platform 12 that has radio-translucent regions to allow image acquisition. An x-ray source 14 with a motorized x-ray collimator 26 directs radiation to a digital detector 16, with the emitted radiation energy represented by a central ray 18. Central ray 18 is preferably orthogonal to the planar incident surface of detector 16. A translation apparatus 20, such as an x-y stage driven by actuator 22, provides motion for suitably positioning detector 16 for receiving incident radiation that is conveyed through and near to the patient anatomy of interest. Detector 16 is in signal communication with a control logic processor 40. Image-bearing signals from detector 16 are sent to control logic processor 40. The signal data content can then be locally rendered on a display 24 that is in signal communication with processor 40. Control logic processor 40 has a memory 44 for storing acquisition parameters and acquired image data content. The image data content can also be transmitted to one or more networked processors 42 for further processing and storage.

A number of sensors can provide information that is needed to support automated positioning of imaging components or, alternately, to provide feedback to control logic processor 40 that guides manual positioning. For example, an inclinometer 28 can be used to sense the relative inclination angle of detector 16. A camera 30 can be used to obtain reflectance images that show the relative alignment of x-ray source 14 to the patient anatomy. Camera 30 images can be used to detect one or more fiducials (not shown), used by processor 40 logic for positioning information. Camera 30 can be a stereoscopic camera, as described previously. An actuator 32 provides angular and x- and y- axis translational movement in the plane of detector 16 (as shown by conventional x, y, and z axes in FIG. 1) for guiding the path of radiation from x-ray source 14 from the x-ray tube.

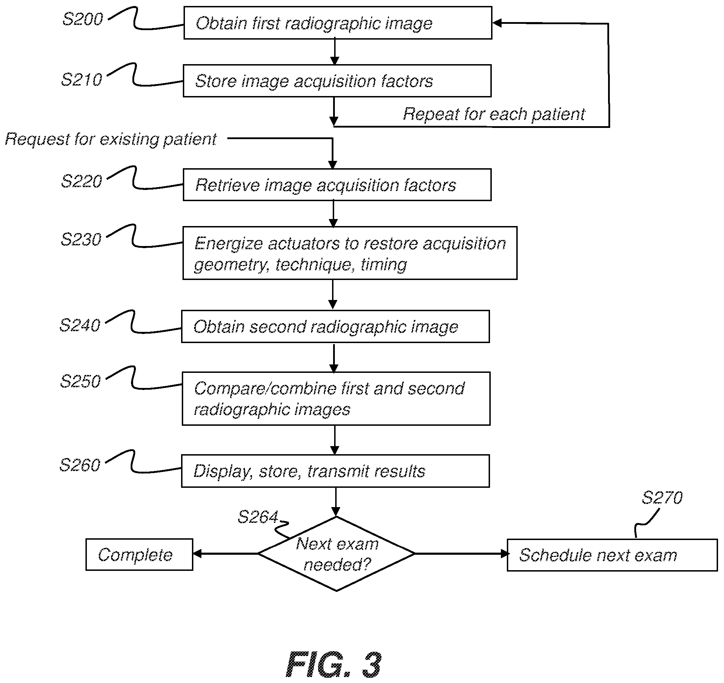

The logic flow diagram of FIG. 3 shows a processing sequence for automated scheduling, maintaining consistent rendering for radiographic images by tracking and controlling various factors that affect image appearance, geometry, and image-to-image registration according to an embodiment of the present disclosure. In an acquisition step S200, a first radiographic image is obtained, having an associated set of acquisition factors. A recording step S210 is executed, in which the set of acquisition factors or parameters used for the first image are recorded in memory as a set of acquisition settings and linked with the obtained first image and with the patient ID. The recorded acquisition factor data is then available for future use. Exemplary acquisition factors that can be recorded as the set of acquisition settings can include the following:
(i) X-ray acquisition geometry, including the various measurements that indicate the relative alignment of the x-ray tube of x-ray source 14 with detector 16 as well as distance between source 14 and detector 16. This can include translation as well as rotation data, for example. Image tube movement for tomosynthesis can also be recorded for matching.
(ii) Patient angular inclination, since this can be related to fluid effects and other complicating factors.
(iii) Image capture technique settings, including set energy (kVp) and intensity (mAs) levels.
(iv) Timing data for the respiratory cycle of the patient, as described subsequently.

In a subsequent imaging session, identification of the patient and type of exam needed can activate a retrieval step S220 in which the recorded acquisition factor data, as noted previously, can be accessed from memory and used to set up and monitor the positioning of the imaging apparatus for acquiring a second radiographic image in a configuration step S230. As was described in the schematic diagram of FIG. 1, the imaging system logic can directly control one or more actuators that automatically set up the system configuration. Operator override of automated setup and monitoring can be provided. Once the system configuration is consistent with the stored settings information, the subsequent image can be obtained in an acquisition step S240. The first and subsequent images can be combined and compared in a processing step S250.

Various types of automated processing can be provided, using programmed or machine learning logic. As just one example of a simplified processing approach, subtraction can be applied, pixel by pixel, in order to ascertain how much difference there is between the images and to show more detailed information on patient condition. Subsequent description provides more detailed information on processing logic and options.

According to an alternate embodiment of the present disclosure, an operator interface on display 24 (FIG. 1) can provide text, graphical, or audible instructions to guide manual adjustment of source and detector position. The operator interface can also provide data for technique settings and other information for use by the operator in configuring the imaging apparatus for obtaining consistent imaging results for subsequent images, based on stored configuration parameter data for previous images. Feedback from sensor detection can help to guide operator adjustment, indicating that dimensional or angular positioning does not match that used for previous exams and suggesting remedial modification, for example. Feedback sensing can be continuous as adjustments are made, in order to help fine-tune the positioning geometry, for example.

The sequence of FIG. 3 can be used for a wide variety of x-ray modalities, including conventional 2D radiographic imaging, tomosynthesis and other volume imaging and depth imaging modes, serial radiography or "video x-ray" imaging. A rendering step S260 appropriately displays or transmits the newly acquired imaging data. In general, embodiments may include using limited angle digital tomosynthesis captured images, serial radiography, general conventional x-ray radiographs, dynamic digital tomosynthesis, and dual energy captured images.

Following results storage in rendering step S260, the sequence of FIG. 3 continues with a verification step S264 that determines whether or not a subsequent exam should be scheduled and determines a suitable time interval. If needed, a scheduling step S270 then schedules a subsequent exam based on the assessment of patient condition. If a subsequent exam is not needed, no further action is taken.

Using the same image technique settings can be particularly useful for obtaining consistency. Differences in these settings can change the appearance of lung disease, such as pneumonia or pulmonary edema, for example. Consistent grayscale presentation can help to identify various patient conditions.

It should be noted that the sequence of FIG. 3 can be assisted using machine-learning logic, as described in more detail subsequently.

### Respiratory cycle

It is widely acknowledged that motion of the anatomy can impact the appearance of the radiographic image and impair the task of image evaluation for the clinician. This can be particularly true for imaging of the chest, where patient movement due to breathing can affect the overall position of the patient and alter the relative relationship of ribs and other bone structures to lungs and other internal organs.

Difficulties such as those encountered in image analysis due to patient breathing, for example, have prompted the development of serial radiography or "video x-ray" techniques. Using serial radiography, a set of images is acquired, with the series of images sequentially obtained spanning the breathing cycle, for example. Serial radiography can be an effective imaging mode for capture of the repeated breathing cycle at successive phases.

In order to make an effective comparison between images taken in first and subsequent sessions, however, it is necessary to match the timing, or phases, of the breathing cycle from each session. Otherwise, comparison can be compromised by differences in relative position of the imaged features. Disparities for images acquired during different phases of the breathing cycle can be particularly difficult for machine-learning logic, complicating the needed computation power for properly compensating for differences in position.

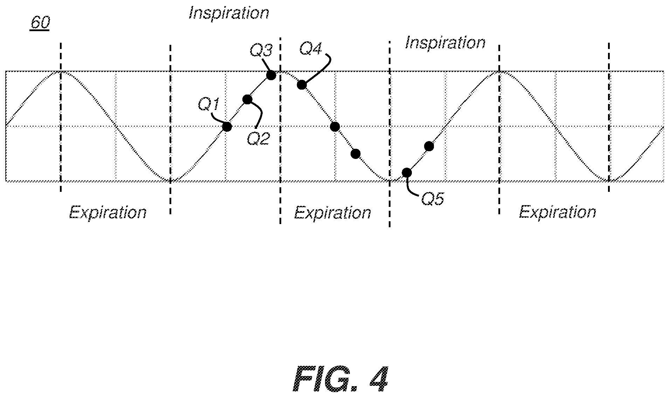

The timing diagram graph 60 of FIG. 4 shows, in simplified form, a representative portion of the normal breathing cycle, with recurring periods of inspiration and expiration. Exemplary instants of the cycle are labeled Q1, Q2, ... Q5 for illustration. It can be appreciated that the appearance and positioning of lung features for images captured at full inspiration, such as those captured with patient breathing at instants Q3 or Q4 for example, would differ from the appearance and positioning for images captured at or near full expiration, such as at instant Q5 in FIG. 4.

To help improve the effectiveness of serial radiography for chest imaging, embodiments of the present disclosure track patient breathing during the radiographic exam and record the relative point in the breathing cycle that corresponds to each image obtained in the set of images obtained in the serial radiography series. Thus, for example, timing data relative to the breathing cycle can be stored for images in a first series in step S210, as described previously with reference to FIG. 3.

For obtaining chest x-ray images in a subsequent session, the relative timing of image captures to the breathing cycle can be tracked and recorded, allowing a close match between serial images of the first set, as described previously, and corresponding images of the subsequent series. Alternately, the timing of subsequent session can be matched to the timing of the first serial session. Thus, for example, acquisition of image content for both the first series of images and the second series of images can be triggered according to the patient breathing pattern, such as at full inspiration, or other suitable trigger condition related to movement of the patient, further allowing the two breathing sequences to be compared, as well as facilitating the comparison of any two images taken at the same phase of the breathing cycle in each of their respective sessions.

Tracking of the patient breathing cycle can be performed using camera 30 (FIG. 1) that obtains reflectance images or a separate camera or sensor. A camera could be used, for example, to monitor patient breathing from the same position. According to an embodiment of the present disclosure, camera 30 can have depth measurement capability, such as that available using a LIDAR camera such as the REALSENSE(TM) LIDAR Camera from Intel(R). Alternately, depth data can be obtained using an RGB-D color depth camera such as the time-of-flight AZURE KINECT DK depth camera from Microsoft. Based on camera images, processor 40 can control timing of single-mode x-ray images as well as timing to initiate serial radiography or correlate individual serial radiography series images. The breathing cycle can be stored as part of the patient record, for example.

Additional aspects may include temporal, grayscale and geometric registration of a more recent captured motion sequence with one or more prior series and leveraging pseudo-invariant features as tie, or anchor, points, i.e., features within the image that remain reasonably (pseudo) invariant to the breathing motion, while registering the other structures that vary in position and grayscale significantly during the breathing cycle.

According to an embodiment of the present disclosure, an ICU room-based imaging system may facilitate the capture of higher dimensional imagery, with greater than 2-dimensions, such as temporal (dynamic) x-ray, spectral (dual energy) imaging, and 3D-spatial (digital tomosynthesis) x-ray capture of critically ill patients in the ICU. Such x-ray imaging is more challenging when using mDR.

According to an embodiment of the present disclosure, a system and/or method for the capture of 2D dynamic x-ray acquisition is disclosed using more than one perspective. For example, dynamic x-ray sequences can be captured from two perspectives---such as AP (anteroposterior) and an oblique view. In one embodiment, the capture of the dynamic sequences is synchronized in time and, as such, the dynamic sequences are synchronized with respect to the phase of the patient's breathing cycle. In order to capture synchronized dynamic x-ray image sequences from two perspectives, two DR detectors and two x-ray sources can be used.

According to an embodiment of the present disclosure, either one detector and x-ray tube or multiple detectors and tubes can be systematically moved about the patient anatomy during image capture in a precise, calibrated manner, such that a greater number of dynamic perspectives are captured during the acquisition sequence. Images captured in this synchronized dynamic sequence can be processed to produce a pseudo-3D motion sequence of the patient, for example, representing the patient's full breathing cycle.

### Automated Analysis and Processing

As noted previously with respect to step S250 in the FIG. 3 sequence, image analysis can be provided by a comparison of images taken in different exam sessions. Automated comparison can be particularly effective where the compared images have been captured using consistent geometry and acquisition settings.

One advantage of solutions described herein relates to improved image suitability for automated processing using machine learning. For example, a deep-learning based analysis of related physiologic imaging exams can be used to compare prior images with more recent images in order to identify a change in a patient's condition (e.g., improved or worsened) and a magnitude of the change. The amount of change in patient condition can be simply expressed, such as numerically rated from 0-10 in a simpler embodiment. Alternately, the relative degree of patient change can be expressed as a more complex data vector that provides multi-dimensional data and patient measurements. A severity of a patient's condition may be quantified, e.g., automatically generating an overall severity score for lung opacification. Prognostics generated from the automated analysis can include probability of needing intubation within the next X number of days, probability of mortality within the next X number of days, or other likely outcomes.

Based on actual patient data obtained over time, machine learning can also be used for providing initial setup of acquisition parameters, given various information about the patient condition. Automated analysis has the advantage of using patient history and other data that may not be easily accessible to the practitioner, in addition to using learned data from imaging other patients.

It can be appreciated that methods described herein can be useful for any of the radiographic modalities, including fluoroscopy, wherein repositioning to align geometry of imaging apparatus and the patient may be particularly important. In particular, methods of the present disclosure can be useful for imaging modalities that require use of a mobile C-arm, such as the Mobile C-arm System from Carestream Health, Rochester, NY, wherein the imaging signal source needs to be moved in and out of the field in order to provide work-space for the attending practitioner.

By improving the consistency of image content, including not only position and geometry of the imaging setup, but also timing and post-processing of the image content, embodiments of the present disclosure simplify the task of providing data for more complex analysis and processing.

### Post-processing of image content

Grayscale correction or normalization can be applied to image content as a tool for providing improved consistency and analysis. As is well known in the imaging arts, grayscale correction can be applied in a number of ways, such as by controlling response curves or other transform mechanisms that are used for mapping digital image values to display values.

As another post-processing operation, rib segmentation may be used for the quantification of respiratory volumes or changes in volumes versus prior images. This quantification method may be used as a proxy for tidal volumes or for musculoskeletal breathing recruitment and/or differences thereof versus prior images.

Processing of the image content from different sessions can include digital subtraction, for example, or other pixel-to-pixel or grouped pixel processing techniques.

### Subsequent Exam Scheduling

Where images of ICU patients have been captured in a consistent manner, it is possible to implement exam triggering in a number of ways. As mentioned previously, a next exam may be automatically triggered according to analyses of one of more prior exams. Analyses may include assessments of changes in anatomical features, or the degree of changes in abnormal disease processes. For example, dramatic changes in lung opacity can indicate increased fluid content. A next exam may also be triggered by an automated program using mathematical models of longitudinally captured prior exams. Triggering of the timing of a next exam, may be either automatically calculated and performed, or may be based on alerts generated by programmed mathematical models, with the actual exam exposure then performed by a radiographic technologist. As an alternative, scheduling of the exam acquisition may default to departmental clinical practice or other standard guidelines.

As was shown and described previously with respect to the sequence shown in FIG. 3, comparison step S250 is a key part of the image processing regimen, executing the longitudinal, or time-dimensioned, analysis that provides useful information for assisting in assessing patient condition and treatment requirements. The imaging system can act upon processing results that indicate suggested scheduling for subsequent exams in a number of ways.

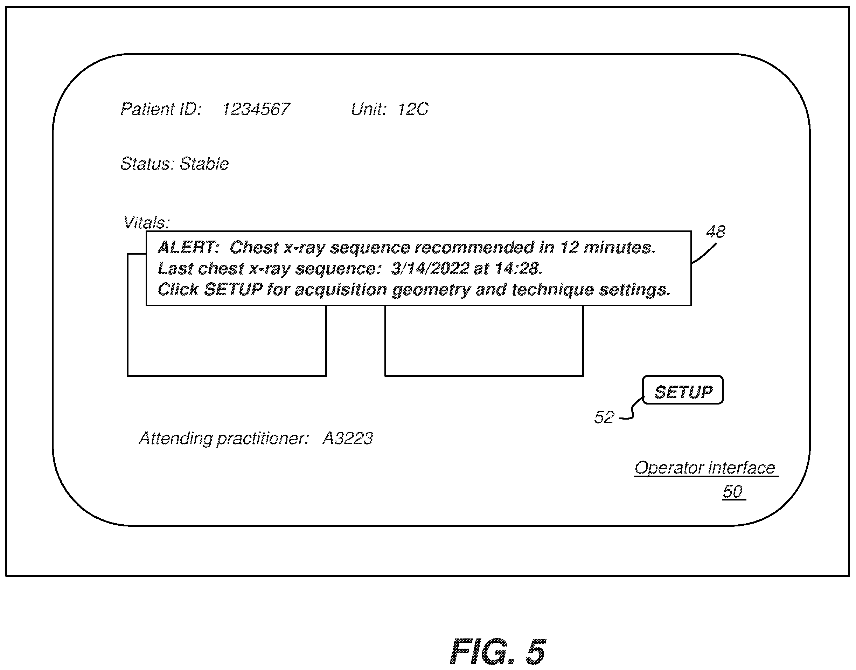

According to an embodiment of the present disclosure, the imaging system provides an automatic scheduling capability that displays an imaging timing recommendation, reporting the needed image type, and prompting the radiography staff or attending practitioner when images are needed. An exemplary operator interface screen 50 in FIG. 5 shows a reminder prompt 48 that can be displayed. Prompt 48 can be rendered in bold, flashing, or otherwise highlighted to catch attention of the staff.

Alternately, the automatic scheduling can generate an entry in a computerized scheduling system that is maintained for the attending staff. An optional Setup button 52 can be displayed, allowing the viewer to display a listing of stored acquisition settings associated with the image, including type of image needed and parameters for consistent acquisition geometry and technique settings. Various types of scheduling software can be executing in the ICU or general hospital environment. Signals provided by the apparatus of the present disclosure can be used to interface with existing scheduling systems and to enter scheduling requests with or without operator intervention, depending on the particular requirements of the medical care facility.

According to an alternate embodiment of the present disclosure, the scheduled image capture can be automatically executed, with set up for imaging performed by the imaging apparatus and approval or other acknowledgement to initiate exposure entered by the attending staff.

Mathematical models to assist in exam scheduling can be generated, in whole or in part, using machine learning techniques. Feature vectors for training and for decision-making can be formed based not only on image comparison, but also including vital signs and patient history data, as well as the set of image acquisition settings as outlined above, for example.

According to an embodiment of the present disclosure, the system can provide an interim, temporal monitoring capability by capturing scout or sampling images, such as projection radiographs at very low dose levels and/or at low spatial resolution. Taken at regular intervals between exams, scout images can also be obtained using consistent image acquisition geometry, consistent technique settings, and, for chest x-rays, with similar timing with respect to the respiratory cycle of the patient.

The low dose or low spatial resolution images can be monitored, either automatically or with human assistance, for gross changes in patient respiratory status, or other changes commensurate with the reduced spatial, signal-to noise, or grayscale resolution imaging. Based on the high temporal sampling of the patient condition, models can be developed that either automatically trigger the appropriate (indicated) high-resolution exam. The high-resolution exam can be, for example, a standard 2D radiograph. The ideal 2D projection can be specified, e.g., AP, oblique, or lateral exposure, a dynamic sequence, a dual energy capture, or a digital tomosynthesis capture. The low dose temporal sampling may be performed from a consistent view projection, a stratified sampling of view projections, or from a randomized sampling of view projections.

A computer program product may include one or more storage medium, for example; magnetic storage media such as magnetic disk (such as a floppy disk) or magnetic tape; optical storage media such as optical disk, optical tape, or machine readable bar code; solid-state electronic storage devices such as random access memory (RAM), or read-only memory (ROM); or any other physical device or media employed to store a computer program having instructions for controlling one or more computers to practice the method according to the present invention.

The invention has been described in detail, and may have been described with particular reference to a suitable or presently preferred embodiment, but it will be understood that variations and modifications can be effected within the scope of the invention. The presently disclosed embodiments are therefore considered in all respects to be illustrative and not restrictive. The scope of the invention is indicated by the appended claims.

## Claims

1. A method comprising:
capturing a plurality of radiographic images (62, 64) of a patient (P), each using a same geometric relationship relative to an x-ray source (14), the patient (P) and a digital detector (16), a same energy and intensity of an x-ray beam emitted by the x-ray source (14) and a same phase of the patient's (P) respiratory cycle (Q1, Q2, ... Q5);
determining a change in the radiographic images (62, 64) of the patient (P) based on the plurality of captured radiographic images (62, 64) of the patient (P); and
automatically scheduling a point in time to capture a next radiographic image (64) of the patient (P), wherein the capture of the next radiographic image (64) is performed using the same geometric relationship;
wherein the same phase of the patient's (P) respiratory cycle (Q1, Q2, ... Q5) is determined using a stereoscopic camera, and
wherein the x-ray source (14) is automatically activated at the automatically scheduled point in time to capture the next radiographic image (64) of the patient (P) at the same phase of the patient's (P) respiratory cycle (Q1, Q2, ... Q5).

2. The method of claim 1, further comprising automatically activating the x-ray source (14) to capture dual energy or 3D volume images of the patient (P) using the same geometric relationship.

3. The method of claim 2, further comprising capturing x-ray image sequences of the patient (P) from two perspectives using two digital radiographic detectors (16) and two x-ray sources (14) simultaneously.

4. The method of claim 3, further comprising moving the two digital radiographic detectors (16) and two x-ray sources (14) simultaneously while capturing a plurality of synchronized dynamic radiographic images (62, 64) of the patient (P).

5. The method of claim 4, further comprising processing the plurality of synchronized dynamic radiographic images (62, 64) of the patient (P) to generate a 3D motion sequence of the patient (P).

6. The method of claim 1, wherein automatically scheduling comprises providing a signal to a scheduling software used for patient (P) care.

7. The method of claim 1, further comprising electronically storing acquisition factors that electromechanically define the same geometric relationship.

8. The method of claim 7, further comprising electronically retrieving the stored acquisition factors to automatically position the x-ray source (14) and the digital detector (16) at the common geometric relationship.

9. The method of claim 8, further comprising electronically accessing the stored acquisition factors to automatically set the same energy and intensity of an x-ray beam emitted by the x-ray source (14) to capture the plurality of radiographic images (62, 64) of the patient (P).

## Patentansprüche

1. Ein Verfahren, das Folgendes aufweist:
Erfassen einer Vielzahl von Röntgenbildern (62, 64) eines Patienten (P), wobei jedes Bild eine gleiche geometrische Beziehung in Bezug auf eine Röntgenquelle (14), den Patienten (P) und einen digitalen Detektor (16), eine gleiche Energie und Intensität eines von der Röntgenquelle (14) emittierten Röntgenstrahls und eine gleiche Phase des Atmungszyklus (Q1, Q2, ... Q5) des Patienten (P) verwendet;
Bestimmen einer Änderung in den Röntgenbildern (62, 64) des Patienten (P) auf Grundlage der Vielzahl von erfassten Röntgenbildern (62, 64) des Patienten (P); und
automatisch Planen eines Zeitpunkts zum Erfassen eines nächsten Röntgenbildes (64) des Patienten (P), wobei die Erfassung des nächsten Röntgenbildes (64) unter Verwendung derselben geometrischen Beziehung durchgeführt wird;
wobei dieselbe Phase des Atmungszyklus (Q1, Q2, ... Q5) des Patienten (P) unter Verwendung einer stereoskopischen Kamera bestimmt wird, und
wobei die Röntgenquelle (14) automatisch zu dem automatisch geplanten Zeitpunkt aktiviert wird, um das nächste Röntgenbild (64) des Patienten (P) in derselben Phase des Atmungszyklus (Q1, Q2, ... Q5) des Patienten (P) aufzunehmen.

2. Das Verfahren nach Anspruch 1, das ferner automatisches Aktivieren der Röntgenquelle (14) aufweist, um Dual-Energy- oder 3D-Volumenbilder des Patienten (P) unter Verwendung derselben geometrischen Beziehung zu erfassen.

3. Das Verfahren nach Anspruch 2, das ferner Erfassen von Röntgenbildsequenzen des Patienten (P) aus zwei Perspektiven aufweist, und zwar unter gleichzeitiger Verwendung von zwei digitalen Röntgendetektoren (16) und zwei Röntgenquellen (14).

4. Das Verfahren nach Anspruch 3, das ferner gleichzeitiges Bewegen der zwei digitalen Röntgendetektoren (16) und der zwei Röntgenquellen (14) aufweist, während eine Vielzahl synchronisierter dynamischer Röntgenbilder (62, 64) des Patienten (P) erfasst wird.

5. Das Verfahren nach Anspruch 4, das ferner Verarbeiten der Vielzahl synchronisierter dynamischer Röntgenbilder (62, 64) des Patienten (P) aufweist, um eine 3D-Bewegungssequenz des Patienten (P) zu erzeugen.

6. Das Verfahren nach Anspruch 1, wobei das automatische Planen Bereitstellen eines Signals an eine Planungssoftware aufweist, die für die Patientenversorgung (P) verwendet wird.

7. Das Verfahren nach Anspruch 1, das ferner elektronisches Speichern von Erfassungsfaktoren aufweist, die dieselbe geometrische Beziehung elektromechanisch definieren.

8. Das Verfahren nach Anspruch 7, das ferner elektronisches Abrufen der gespeicherten Erfassungsfaktoren aufweist, um die Röntgenquelle (14) und den digitalen Detektor (16) automatisch in der gemeinsamen geometrischen Beziehung zu positionieren.

9. Das Verfahren nach Anspruch 8, das ferner elektronisch Zugreifen auf die gespeicherten Erfassungsfaktoren aufweist, um automatisch dieselbe Energie und Intensität eines von der Röntgenquelle (14) emittierten Röntgenstrahls einzustellen, um die Vielzahl von Röntgenbildern (62, 64) des Patienten (P) aufzunehmen.

## Revendications

1. Procédé comprenant :
la capture d'une pluralité d'images radiographiques (62, 64) d'un patient (P), en utilisant à chaque fois une même relation géométrique par rapport à une source de rayons X (14), au patient (P) et à un détecteur numérique (16), de mêmes énergie et intensité d'un faisceau de rayons X émis par la source de rayons X (14) et une même phase du cycle respiratoire (Q1, Q2, **...** Q5) du patient (P) ;
la détermination d'une modification dans les images radiographiques (62, 64) du patient (P) sur la base de la pluralité d'images radiographiques capturées (62, 64) du patient (P) ; et
la planification automatique d'un moment pour capturer une image radiographique suivante (64) du patient (P), dans lequel la capture de l'image radiographique suivante (64) est effectuée en utilisant la même relation géométrique ;
dans lequel la même phase du cycle respiratoire (Q1, Q2, **...** Q5) du patient (P) est déterminée en utilisant une caméra stéréoscopique, et
dans lequel la source de rayons X (14) est activée automatiquement au moment programmé automatiquement pour capturer l'image radiographique suivante (64) du patient (P) à la même phase du cycle respiratoire (Q1, Q2, **...** Q5) du patient (P).

2. Procédé selon la revendication 1, comprenant en outre l'activation automatique de la source de rayons X (14) pour capturer des images à double énergie ou en volume 3D du patient (P) en utilisant la même relation géométrique.

3. Procédé selon la revendication 2, comprenant en outre la capture de séquences d'images radiographiques du patient (P) à partir de deux perspectives en utilisant deux détecteurs radiographiques numériques (16) et de deux sources de rayons X (14) en même temps.

4. Procédé selon la revendication 3, comprenant en outre le déplacement des deux détecteurs radiographiques numériques (16) et des deux sources de rayons X (14) en même temps tout en capturant une pluralité d'images radiographiques dynamiques synchronisées (62, 64) du patient (P).

5. Procédé selon la revendication 4, comprenant en outre le traitement de la pluralité d'images radiographiques dynamiques synchronisées (62, 64) du patient (P) pour générer une séquence de mouvement 3D du patient (P).

6. Procédé selon la revendication 1, dans lequel la planification automatique comprend la fourniture d'un signal à un logiciel de planification utilisé pour soigner le patient (P).

7. Procédé selon la revendication 1, comprenant en outre le stockage électronique de facteurs d'acquisition qui définissent électro-mécaniquement la même relation géométrique.

8. Procédé selon la revendication 7, comprenant en outre la récupération électronique des facteurs d'acquisition stockés pour positionner automatiquement la source de rayons X (14) et le détecteur numérique (16) selon la même relation géométrique.

9. Procédé selon la revendication 8, comprenant en outre l'accès électronique aux facteurs d'acquisition stockés pour régler automatiquement les mêmes énergie et intensité d'un faisceau de rayons X émis par la source de rayons X (14) pour capturer la pluralité d'images radiographiques (62, 64) du patient (P) .
